# FASCICULE DE BREVET EUROPEEN

(11) **EP 3 986 245 B1**
(45) Date de publication et mention de la délivrance du brevet: **03.09.2025**
(21) Numéro de dépôt: 20736257.5
(22) Date de dépôt: 19.06.2020
(51) Int. Cl.: A61B 3/103, A61B 3/10

(54) **PROCEDE DE MESURE DE LA CONVERGENCE DES YEUX D'UN PATIENT**
VERFAHREN ZUR MESSUNG DER KONVERGENZ DER AUGEN EINES PATIENTEN
METHOD FOR MEASURING THE CONVERGENCE OF THE EYES OF A PATIENT

(30) Priorité: 21.06.2019 FR 1906698
(43) Date de publication de la demande: 27.04.2022
(73) Titulaire: Eyesoft, 33800 Bordeaux (FR)
(72) Inventeur: DIDIER, Thomas, 33140 Villenave d'Ornon (FR); PERSILLON, Audrey, 33800 Bordeaux (FR)
(74) Mandataire: Aquinov
(86) Numéro de dépôt international: PCT/EP2020/067067
(87) Numéro de publication internationale: WO 2020/254555

(56) Documents cités:
- EP-A1- 3 018 523
- KR-A- 20160 147 636
- US-A1- 2009 153 796
- US-A1- 2019 180 437

## Description

L'invention concerne le domaine de l'orthoptie. Plus spécifiquement, l'invention concerne le domaine de l'évaluation de troubles de la vision binoculaire dans le cadre d'un examen oculomoteur d'un patient.

Un examen oculomoteur d'un patient a notamment pour but de mettre en évidence une incapacité du patient à loucher de façon satisfaisante, traduisant un trouble de convergence dû à un déséquilibre oculomoteur et pouvant provoquer une asthénopie (fatigue visuelle, maux de têtes, vision floue ou double, larmoiement). Afin de réaliser cet examen, il est connu de procéder à un test de convergence consistant à approcher une baguette de fixation en direction des yeux du patient pour provoquer une demande vergentielle et d'observer le mouvement des yeux au fur et à mesure du mouvement de la baguette afin de vérifier que la convergence des yeux est symétrique et simultanée. Ce test n'est pas satisfaisant car il dépend entièrement du praticien, lequel peut être déconcentré, ce qui pourrait fausser la manipulation de la baguette ainsi que rendre imprécise l'observation du mouvement des yeux et donc le diagnostic.

En outre, dans le cas d'un diagnostic d'un défaut de convergence nécessitant une rééducation des yeux, ce procédé doit être renouvelé au cours du temps pour procéder à cette rééducation. Or, il a été constaté que ce procédé n'est pas suffisamment stimulant et peut amener le patient à abandonner les séances de rééducation.

D'autres procédés sont également connus, comme par exemple l'emploi d'une barrette de prismes ou encore d'un synoptophore. Ces procédés présentent les mêmes inconvénients car ils nécessitent, d'une part, le mouvement mécanique d'un objet pour provoquer la demande vergentielle et, d'autre part, seule une réponse subjective du patient ou une observation par le praticien des yeux du patient informe sur l'état de convergence du patient. D'autres dispositifs et procédés de mesure objective sont aussi connus, par exemples dans les documents EP 3 018 523 A1 (THALES SA) ou US 2019/180437 A1 (MANELA ISRAEL) .

La présente invention a ainsi pour objet de pallier ces inconvénients, en proposant un procédé de mesure de la convergence des yeux d'un patient objectif, qui ne soit pas dépendant de la réponse du patient, du jugement du praticien et qui maintienne l'intérêt du patient au cours de sa rééducation.

L'invention a ainsi pour objet un procédé de mesure de la convergence des yeux d'un patient mis en œuvre par un dispositif comportant un casque de réalité virtuelle et un oculomètre, le procédé comportant les étapes suivantes :
a. on affiche sur un afficheur du casque une animation réalisant un effet de jaillissement d'un objet vers le patient;
b. on mesure pendant l'affichage de l'animation, au moyen de l'oculomètre, la direction du regard de chaque œil du patient ;
c. on calcule, à partir de chaque direction du regard mesurée, une valeur de convergence pour chaque œil du patient.

On comprend que l'emploi, d'une part, d'un casque de réalité virtuelle et, d'autre part, d'un oculomètre (également appelé en anglais Eye-tracker ou Gaze-tracker) permet de rendre le procédé de mesure de la convergence indépendant de la concentration du praticien. En effet, d'une part, l'affichage par l'afficheur du casque de l'animation réalisant un effet de jaillissement de l'objet vers le patient provoque une demande vergentielle en convergence des yeux sans intervention nécessaire de la part du praticien. D'autre part, l'utilisation d'un oculomètre permet l'observation en temps réel de la réponse en convergence des yeux de façon automatique et fiable, sans que les variations de concentration du praticien ne viennent fausser cette observation. En outre, l'emploi d'une technologie de réalité virtuelle rend le procédé ludique, ce qui maintient l'intérêt du patient éveillé au cours de la rééducation.

On entend par casque de réalité virtuelle tout dispositif destiné à être porté sur la tête du patient et permettant de l'immerger dans une réalité virtuelle. Un tel casque pourra par exemple être équipé d'un visiocasque (ou HMD de l'anglais head-mounted display) stéréoscopique. Le cas échéant, le casque pourra comporter un afficheur unique pour les deux yeux, ou au contraire deux afficheurs distincts, chacun destiné à l'un des yeux du patient. On entend par oculomètre tout dispositif permettant de détecter un mouvement oculaire et de mesurer la direction du regard. Il pourra par exemple s'agir d'une caméra, notamment infrarouge, associée à un calculateur, qui peut être ou non intégrée au casque de réalité virtuelle.

L'étape d'affichage de l'animation comporte selon l'invention l'affichage d'un objet virtuel en 3 dimensions en mouvement selon un axe orthogonal au casque et dirigé vers le visage, et notamment l'arête du nez, du patient. Le cas échéant, l'affichage de l'objet virtuel en 3 dimensions est réalisé par stéréoscopie. Selon un exemple, l'affichage de l'animation pourra comporter l'affichage d'un objet avec parallaxe, notamment une sphère, ou d'un objet sans parallaxe, notamment un carré, pourvu d'une mire centrale, ledit objet se déplaçant depuis une position distante des yeux, par exemple 4 mètres, vers une position proche des yeux, par exemple 5 centimètres du nez.

L'affichage de l'objet virtuel en mouvement est réalisé selon la présente invention avec une vitesse décroissante selon que l'objet se rapproche du visage du patient. Le pas de déplacement de l'objet peut ainsi être plus grand lorsque l'objet est éloigné que lorsqu'il est proche, de sorte à obtenir une mesure de la direction du regard de chaque œil précise lorsque l'objet est proche.

Avantageusement, chaque mesure de la direction du regard de chaque œil du patient pendant l'affichage de l'animation au moyen de l'oculomètre comprend la détermination d'un déplacement latéral de cet œil par rapport à l'axe orthogonal du mouvement de l'objet virtuel en trois dimensions. L'étape de mesure permet ainsi d'obtenir un échantillon composé d'une pluralité de directions du regard associées chacune à une distance distincte de l'objet affiché à l'œil.

Avantageusement encore, chaque direction du regard de chaque œil mesurée par l'oculomètre est déterminée sous la forme d'un vecteur direction dans un espace en trois dimensions et la valeur de convergence est calculée à partir de la projection dudit vecteur direction sur un plan sensiblement horizontal. En effet, la convergence des yeux étant un phénomène s'exprimant de façon horizontale, cette caractéristique permet d'obtenir des valeurs de convergence cohérentes.

Selon un mode de réalisation de l'invention, le procédé comporte une étape préalable de calibration dans laquelle on détermine un vecteur direction d'origine du regard du patient. Cette étape préalable pourra par exemple comprendre l'affichage d'un objet virtuel sur l'afficheur du casque de réalité virtuelle, l'objet virtuel étant placé à une distance correspondant à l'infini physiologique pour déterminer ledit vecteur direction d'origine du regard. En outre, l'étape préalable de calibration pourra comprendre l'animation de cet objet virtuel selon un mouvement donné, de sorte à permettre à l'oculomètre de détecter la pupille de chaque œil et ainsi de rendre la mesure de la direction du regard de chaque œil fiable.

Avantageusement, chaque valeur de convergence, pour chaque œil, est calculée par comparaison du vecteur direction du regard de cet œil mesurée et dudit vecteur direction d'origine. En variante, chaque valeur de convergence, pour chaque œil, est calculée par comparaison de la direction du regard de cet œil mesurée et un axe horizontal. Le cas échéant, la comparaison pourra comprendre le calcul d'un angle séparant chaque direction du regard de l'œil mesurée et la direction d'origine ou l'axe horizontal. Si on le souhaite, chaque valeur de convergence correspondra audit angle, calculé en radian. En variante, chaque valeur de convergence pourra correspondre à une conversion dudit angle en valeur dioptrique.

Avantageusement, le procédé peut comporter une étape d'affichage sur une interface graphique d'une courbe de l'évolution des valeurs de convergence calculées pour chaque œil en fonction de la distance de l'objet au patient. Si on le souhaite, le procédé peut comporter une étape de diagnostic d'un défaut de convergence en fonction des valeurs de convergence calculées, par exemple mettant en œuvre un algorithme de type réseau neuronal dont les poids synaptiques et/ou les valeurs de biais de la ou des couches du réseau neuronal ont été déterminés à l'aide d'échantillons de paires valeur de convergence/distance de l'objet au patient préétablis et pour lesquels un défaut de convergence est connu.

L'invention a également pour objet un programme d'ordinateur comprenant un code de programme qui est conçu pour mettre en œuvre le procédé selon l'invention.

L'invention a également pour objet un support de données sur lequel est enregistré le programme d'ordinateur selon l'invention.

La présente invention est maintenant décrite à l'aide d'exemples uniquement illustratifs et nullement limitatifs de la portée de l'invention, et à partir des illustrations jointes, dans lesquelles :
[Fig. 1] représente un procédé de mesure de la convergence des yeux d'un patient selon un mode de réalisation de l'invention ;
[Fig. 2] représente une étape de calibration du procédé de mesure selon la [Fig. 1] ;
[Fig. 3] représente d'autres étapes du procédé de mesure selon la [Fig. 1] ;
[Fig. 4] représente une courbe d'évolution des valeurs de convergence pour un premier patient obtenues à l'issue des étapes de la [Fig. 3] ; et
[Fig. 5] représente une courbe d'évolution des valeurs de convergence pour un deuxième patient obtenues à l'issue des étapes de la [Fig. 3].

Dans la description qui suit, les éléments identiques, par structure ou par fonction, apparaissant sur différentes figures conservent, sauf précision contraire, les mêmes références.

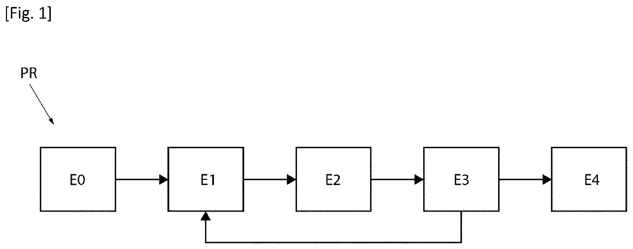

On a représenté en [Fig. 1] un procédé PR de mesure de la convergence des yeux d'un patient selon un mode de réalisation de l'invention. Ce procédé est mis en œuvre au moyen d'un dispositif comportant un casque de réalité virtuelle et d'un oculomètre.

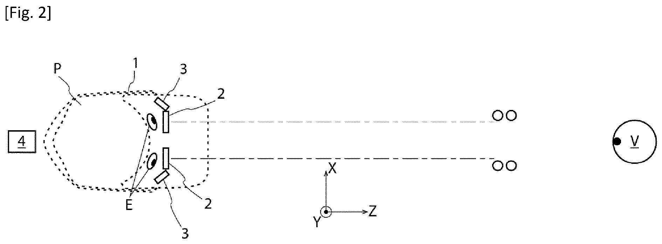

Le procédé 1 comporte une première étape E0 de calibration de l'oculomètre. Cette étape E0 a été représentée en vue de dessus en [Fig. 2]. Lors de cette étape E0, le casque de réalité virtuelle 1 est positionné sur la tête du patient P. Ce casque 1 comporte un afficheur stéréoscopique 2 double disposé en face des yeux E du patient P, et un oculomètre 3, comportant deux caméras orientées chacune en direction d'un des yeux E du patient P. Grâce au casque que de réalité virtuelle 1, le patient P est immergé dans une réalité virtuelle, tandis que l'oculomètre 3 permet de détecter les mouvements des yeux E du patient et de déterminer l'amplitude des rotations de chaque œil selon trois axes vertical Y, horizontal Z et latéral X afin de pouvoir mesurer la direction du regard de cet œil dans l'espace X,Y,Z.

Lors de l'étape E0 de calibration, un objet virtuel V avec parallaxe, dans le cas présent, une sphère présentant une mire en son centre, est affiché, par le biais de l'afficheur 2, en étant placé dans la scène de réalité virtuelle à une distance suffisamment lointaine du patient P pour qu'elle corresponde à l'infini, et le patient est informé qu'il doit regarder cet objet V. L'oculomètre mesure ainsi la direction du regard de chaque œil E afin de déterminer un vecteur de direction d'origine du regard du patient P dans l'espace X,Y,Z. En outre, l'objet virtuel V est déplacé dans la scène de réalité virtuelle afin que l'oculomètre 3 puisse détecter la pupille de chaque œil E et que la mesure de la direction du regard soit la plus fiable possible.

Un calculateur 4 du dispositif projette le vecteur de direction d'origine dans le plan X,Z afin d'obtenir une direction d'origine du regard 00 pour chaque œil E.

Dans une étape E1, l'objet V est placé à une distance donnée d1, par exemple 4 mètres, des yeux Y du patient P. Dans une étape E2, la direction du regard pour chaque œil E est de nouveau mesurée par l'oculomètre 3 sous la forme d'un vecteur direction dans l'espace X,Y,Z. Le calculateur 4 projette ce vecteur direction dans le plan X,Z pour déterminer une direction du regard 01 pour chaque œil E. Dans une étape E3, le calculateur 4 compare ensuite, pour chaque œil E, la direction du regard mesurée 01 avec la direction d'origine 00 pour déterminer un angle α1 séparant ces deux directions. L'angle α1 est stocké dans une mémoire du calculateur en étant appairé à la distance à laquelle l'objet V est placé.

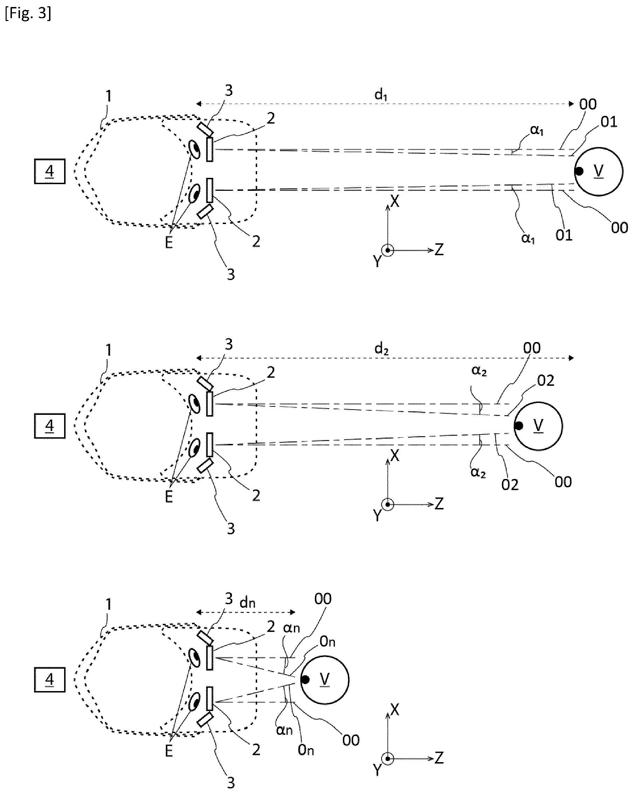

Puis, dans une nouvelle étape E1, l'objet V est déplacé depuis la position précédente vers une nouvelle position, à une distance d2 plus proche des yeux E. Les étapes E2 et E3 sont de nouveau mises en œuvre, de sorte à obtenir un nouvel angle appairé à une nouvelle distance de l'objet V. Les étapes E1 à E3 sont ainsi renouvelées en déplaçant progressivement l'objet V pour le rapprocher des yeux E du patient P jusqu'à ce qu'il soit à une distance dN des yeux. On ainsi représenté en [Fig. 3] plusieurs vues de dessus des étapes E1 à E3 à différents instants du procédé, à savoir lorsque l'objet V est dans sa position la plus éloignée des yeux E, par exemple à une distance d1 de 4 mètres ; lorsque l'objet V est dans une position intermédiaire, par exemple à une distance d2 de 3 mètres, et lorsque l'objet V est dans sa position la plus proche des yeux, par exemple à une distance dN de 5 centimètres. Chacune de ces vues montrent en outre les directions du regard 01, 02 et ON mesurées pour chaque œil E lorsque l'objet V est placé respectivement aux distances d1, d2 et dN, ainsi que les angles α1, α2 et αN obtenus par comparaison de ces directions mesurées avec la direction d'origine 00.

On constate que le déplacement de l'objet V lors des étapes E1 successives est un déplacement selon un axe orthogonal au casque 1 dirigé vers le nez du patient P, qui forme ainsi une animation réalisant un effet de jaillissement de l'objet V vers le patient. Cette animation provoque une demande vergentielle en convergence des yeux E, forçant le patient P à loucher au fur et à mesure que l'objet O se rapproche du nez du patient. Le procédé permet ainsi d'obtenir un échantillon d'angles α1-αN correspondant aux valeurs de convergence pour chaque œil E, obtenus pour différentes distances séparant l'objet O du patient P. Le pas de déplacement de l'objet O diminue au fur et à mesure que la distance entre l'objet O et le patient P diminue, de sorte à obtenir plus de valeurs de convergence lorsque l'objet O est proche du patient et donc que la demande vergentielle en convergence est importante.

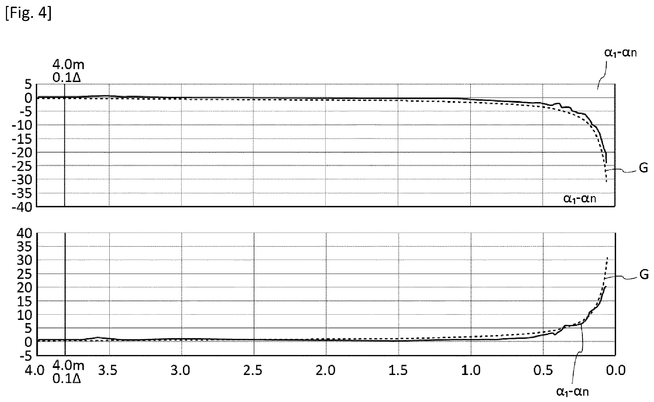

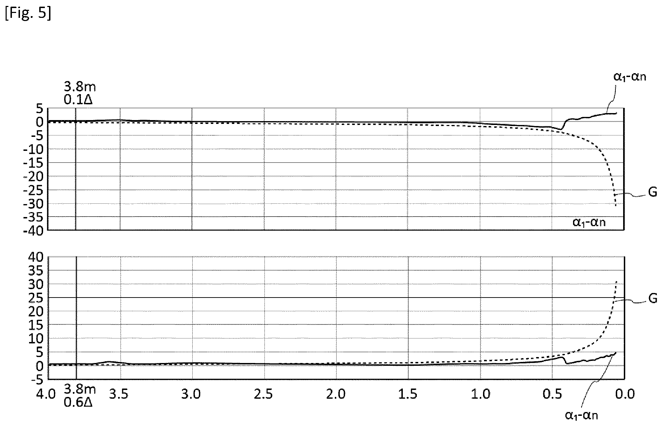

Dans une étape E4, à l'issu de la dernière étape E3, le calculateur affiche sur une interface graphique une courbe de l'évolution des valeurs de convergences calculées pour chaque œil, à savoir l'échantillon d'angles α1-αN transformés en valeurs dioptriques, en fonction des distances de l'objet O au patient P appairées à cet échantillon. L'affichage de ces courbes permet au praticien de procéder à un diagnostic de la capacité de convergence du patient P. On a ainsi représenté en [Fig. 4] les courbes d'évolution des valeurs de convergence d'un premier patient ne souffrant pas ou peu d'un défaut de convergence et en [Fig. 5] les courbes d'évolution d'un second patient souffrant d'un défaut de convergence. On constate ainsi que l'évolution des valeurs de convergence du premier patient est continue et correspond à un gabarit G, tandis que celle du second patient présente un décrochage non conforme au gabarit G et caractérisant un défaut de convergence.

La description qui précède explique clairement comment l'invention permet d'atteindre les objectifs qu'elle s'est fixée, et notamment en proposant un procédé de mesure de la convergence des yeux d'un patient qui, grâce à l'utilisation d'un casque de réalité virtuelle et d'un oculomètre, permet de déterminer une évolution des valeurs de convergence de chaque œil, sans nécessiter d'intervention manuelle du praticien ou d'observation directe des mouvements des yeux, et garde l'intérêt du patient éveillé.

En tout état de cause, l'invention ne saurait se limiter aux modes de réalisation spécifiquement décrits dans ce document, et s'étend en particulier à tous moyens équivalents et à toute combinaison techniquement opérante de ces moyens. On pourra notamment employer tout autre type d'objet virtuel à la place d'une sphère, et notamment un objet sans parallaxe comme un carré.

## Revendications

1. Procédé (PR) de mesure de la convergence des yeux (E) d'un patient (P) mis en œuvre par un dispositif comportant un casque de réalité virtuelle (1) et un oculomètre (3), le procédé comportant les étapes suivantes :
a. (E1) on affiche sur un afficheur (2) du casque une animation réalisant un effet de jaillissement d'un objet (V) vers le patient ;
b. (E2) on mesure pendant l'affichage de l'animation, au moyen de l'oculomètre, la direction du regard (01,02,0N) de chaque œil du patient ;
c. on calcule, à partir de chaque direction du regard mesurée, une valeur de convergence (α1,α2,αN) pour chaque œil du patient
dans lequel l'étape d'affichage (E1) de l'animation comporte l'affichage d'un objet virtuel (V) en trois dimensions en mouvement selon un axe orthogonal au casque (1) et dirigé vers le visage du patient (P) et **caractérisé en ce que** l'affichage de l'objet virtuel (V) en mouvement est réalisé avec une vitesse décroissante selon que l'objet se rapproche du visage du patient (P).

2. Procédé (PR) selon la revendication précédente, dans lequel chaque direction du regard (O1,O2,ON) de chaque œil (E) mesurée par l'oculomètre (3) est déterminée sous la forme d'un vecteur direction dans un espace en trois dimensions (X,Y,Z), et dans lequel la valeur de convergence (α1,α2,αN) est calculée à partir de la projection dudit vecteur direction sur un plan sensiblement horizontal (X,Z).

3. Procédé (PR) selon l'une des revendications précédentes, le procédé comportant une étape préalable de calibration (E0) dans laquelle on détermine un vecteur direction d'origine (OO) du regard du patient (P).

4. Procédé (PR) selon la revendication précédente, dans lequel chaque valeur de convergence (α1,α2,αN) pour chaque œil (E), est calculée par comparaison du vecteur direction du regard (O1,O2,ON) de cet œil mesurée et du vecteur direction d'origine (00).

5. Procédé selon l'une des revendications précédentes, le procédé comportant une étape d'affichage (E4) sur une interface graphique d'une courbe de l'évolution des valeurs de convergence (α1,α2,αN) calculées pour chaque œil (E) en fonction de la distance (d1,d2,dN) de l'objet (V) au patient (P).

6. Programme d'ordinateur comprenant un code de programme qui est conçu pour mettre en œuvre le procédé (PR) selon l'une des revendications 1 à 5 lorsque ledit programme est exécuté par un dispositif comportant un casque de réalité virtuelle (1), un oculomètre (3) et un calculateur (4).

7. Support de données sur lequel est enregistré le programme d'ordinateur selon la revendication 6.

## Patentansprüche

1. Verfahren (PR) zum Messen der Konvergenz von Augen (E) eines Patienten (P), das durch eine Vorrichtung implementiert wird, die ein Virtual-Reality-Headset (1) und einen Eye-Tracker (3) aufweist, wobei das Verfahren die folgenden Schritte aufweist:
a. (E1) Anzeigen auf einer Anzeige (2) des Headsets einer Animation, die einen Effekt eines auf den Patienten zuschießenden Objekts (V) ausführt;
b. (E2) Messen während der Anzeige der Animation mittels des Eye-Trackers der Blickrichtung (O1,O2,ON) jedes Auges des Patienten;
c. Berechnen, aus jeder gemessenen Blickrichtung, eines Konvergenzwerts (α1,α2,αN) für jedes Auge des Patienten
wobei der Schritt des Anzeigens (E1) der Animation die Anzeige eines dreidimensionalen virtuellen Objekts (V) aufweist, das sich entlang einer Achse orthogonal zu dem Headset (1) bewegt und auf das Gesicht (P) des Patienten gerichtet ist, und **dadurch gekennzeichnet, dass** die Anzeige des virtuellen Objekts (V), das sich bewegt, mit einer abnehmenden Geschwindigkeit ausgeführt wird, wenn sich das Objekt dem Gesicht des Patienten (P) nähert.

2. Verfahren (PR) nach dem vorstehenden Anspruch, wobei jede Blickrichtung (O1,O2,ON) jedes Auges (E), die durch den Eye-Tracker (3) gemessen wird, in Form eines Richtungsvektors in einem dreidimensionalen Raum (X,Y,Z) bestimmt wird und wobei der Konvergenzwert (α1,α2,αN) aus der Projektion des Richtungsvektors auf eine im Wesentlichen horizontale Ebene (X,Z) berechnet wird.

3. Verfahren (PR) nach einem der vorstehenden Ansprüche, wobei das Verfahren einen vorangehenden Kalibrierungsschritt (E0) umfasst, in dem ein Vektor einer ursprünglichen Richtung (OO) des Blicks (P) des Patienten bestimmt wird.

4. Verfahren (PR) nach dem vorstehenden Anspruch, wobei jeder Konvergenzwert (α1,α2,αN) für jedes Auge (E) durch einen Vergleich des Vektors der gemessenen Blickrichtung (O1,O2,ON) dieses Auges und des Vektors der ursprünglichen Richtung (OO) berechnet wird.

5. Verfahren nach einem der vorstehenden Ansprüche, wobei das Verfahren einen Schritt zum Anzeigen (E4) einer Kurve der Entwicklung der Konvergenzwerte (α1,α2,αN), die für jedes Auge (E) berechnet werden, auf einer grafischen Schnittstelle in Abhängigkeit der Entfernung (d1,d2,dN) des Objekts (V) zu dem Patienten (P) aufweist.

6. Computerprogramm, umfassend einen Programmcode, der zum Implementieren des Verfahrens (PR) nach einem der Ansprüche 1 bis 5 ausgelegt ist, wenn das Programm durch eine Vorrichtung ausgeführt wird, die ein Virtual-Reality-Headset (1), einen Eye-Tracker (3) und einen Rechner (4) aufweist.

7. Datenträger, auf dem das Computerprogramm nach Anspruch 6 gespeichert ist.

## Claims

1. Method (PR) for measuring the convergence of the eyes (E) of a patient (P), which method is implemented by a device comprising a virtual reality headset (1) and an oculometer (3), the method comprising the following steps:
a. (E1) an animation showing an object (V) appearing to shoot towards the patient is displayed on a display (2) of the headset;
b. (E2) while the animation is being displayed, the gaze direction (O1,O2,ON) of each eye of the patient is measured using the oculometer;
c. a convergence value (α1,α2,αN) for each eye of the patient is calculated from each measured gaze direction
the step (E1) of displaying the animation comprising displaying a three-dimensional virtual object (V) moving along an axis orthogonal to the headset (1) and directed towards the face of the patient (P) and **characterized in that** the moving virtual object (V)
is displayed at decreasing speed as the object moves closer to the face of the patient (P).

2. Method (PR) according to the preceding claim, wherein each gaze direction (O1,O2,ON) of each eye (E) measured by the oculometer (3) is determined as a direction vector in a three-dimensional space (X,Y,Z), and wherein the convergence value (α1,α2,αN) is calculated from the projection of said direction vector onto a substantially horizontal plane (X,Z).

3. Method (PR) according to either of the preceding claims, the method comprising a prior calibration step (E0) in which an origin direction vector (00) of the gaze of the patient (P) is determined.

4. Method (PR) according to the preceding claim, wherein each convergence value (α1,α2,αN) for each eye (E) is calculated by comparing the gaze direction vector (O1,O2,ON) of that eye measured with the origin direction vector (OO).

5. Method according to any of the preceding claims, the method comprising a step (E4) of displaying on a graphic interface a curve of the evolution of the convergence values (α1,α2,αN) calculated for each eye (E) as a function of the distance (d1,d2,dN) from the object (V) to the patient (P).

6. Computer program comprising program code which is designed to implement the method (PR) according to any of claims 1 to 5 when said program is executed by a device comprising a virtual reality headset (1), an oculometer (3) and a computer (4).

7. Data carrier on which the computer program according to claim 6 is saved.
